# EUROPÄISCHE PATENTSCHRIFT

(11) **EP 1 681 076 B1**
(45) Veröffentlichungstag und Bekanntmachung des Hinweises auf die Patenterteilung: **30.04.2008**
(21) Anmeldenummer: 06000387.8
(22) Anmeldetag: 10.01.2006
(51) Int. Cl.: A61M 25/02

(54) **Fixiervorrichtung zum Befestigen eines in eine Körperöffnung einbringbaren vorzugsweise schlauchförmigen medizinischen Hilfmittels**
Fixation means for fixating a preferably tubular medical accessory introducible into a body cavity
Outil de fixation d'un accessoire médical de préférence tubulaire apte à être introduit dans une cavité corporelle

(30) Priorität: 14.01.2005 AT 522005
(43) Veröffentlichungstag der Anmeldung: 19.07.2006
(73) Patentinhaber: Leonh. Lang, 6020 Innsbruck (AT)
(72) Erfinder: Roediger, Claudia, 30938 Thönse (DE); Roediger, Frank, 30900 Wedemark (DE); Ohnmacht, Roland, 6020 Innsbruck (AT)
(74) Vertreter: Torggler, Paul Norbert

(56) Entgegenhaltungen:
- US-A- 3 924 636
- US-A- 5 221 265
- US-A- 5 743 885

## Beschreibung

Die Erfindung betrifft eine Fixiervorrichtung zum Befestigen eines in eine Körperöffnung einbringbaren vorzugsweise schlauchförmigen medizinischen Hilfsmittels an der Haut eines Subjektes.

In der Medizin besteht die Notwendigkeit, Kanülen, Tuben, Schläuche und ähnliche Objekte, die in eine Körperöffnung eines Patienten eingebracht werden, schnell und einfach am Körper zu befestigen und zu fixieren. Insbesondere schlauchförmige medizinische Hilfsmittel, die im Wesentlichen senkrecht aus der Körperoberfläche des Patienten austreten, sollten dabei eine Krümmung erfahren bzw. umgelenkt werden, sodass sie im Wesentlichen parallel zur Körperoberfläche von der Austrittsstelle weggeführt werden.

Es ist bekannt, diese Fixierung der medizinischen Hilfsmittel mithilfe von Pflastern zu bewerkstelligen, die üblicherweise einfache, flächige Klebestreifen sind, die aüs einem Trägermaterial, das einseitig mit einer Klebstoffschicht beschichtet ist, bestehen. US 5 743 885 offenbart ein solches Pflaster zur Positionierung eines medizinischen Hilfsmittels. Weiters sind bereits Fixiervorrichtungen bekannt, die eine Krümmung des medizinischen Hilfsmittels ermöglichen. Derartige bekannte Fixiervorrichtungen verfügen beispielsweise über eine Abstützkomponente mit einer Führungsrinne für das medizinische Hilfsmittel.

Bei den bekannten Fixiervorrichtungen, die von einem flächigen Klebestreifen gebildet sind, besteht die Gefahr, dass das medizinische Hilfsmittel in einer falschen Position oder unter Zug bzw. unter Druck oder mit einer ungünstigen Abknickung fixiert wird, während bei Fixiervorrichtungen mit einer Abstützkomponente mit einer Führungsrinne die geeignete Ausrichtung der Führungsrinne bereits vor dem Aufkleben festgelegt werden muss und die Abmessungen der Führungsrinne dem Durchmesser des schlauförmigen Hilfsmittels annähernd entsprechen müssen. Insgesamt ist die Applikation der bisher: bekannten Fixiervorrichtungen zeitaufwändig und umständlich und daher teuer.

Die vorliegende Erfindung hat es sich daher zur Aufgabe gemacht, eine Fixiervorrichtung zum Befestigen eines in eine Körperöffnung einbringbaren medizinischen Hilfsmittels so auszugestalten, dass die Fixiervorrichtung unter Vermeidung der genannten Nachteile einfach, flexibel und schnell angewendet werden kann, um verschiedenartige Objekte sicher in der korrekten Lage zu fixieren.

Erfindungsgemäß wird dies durch eine Fixiervorrichtung erreicht, die ein auf seiner im Gebrauchszustand der Fixiervorrichtung der Haut zugewandten Unterseite zumindest bereichsweise klebend ausgebildetes Trägerelement mit einer länglichen Durchtrittsöffnung und wenigstens zwei Mittel zum Positionieren des medizinischen Hilfsmittels relativ zum Trägerelement aufweist, wobei die beiden Mittel in Bezug auf die längliche Durchtrittsöffnung gegen überliegend in den End bereichen bezüglich der Längsachse der Durchtrittsöffnung angeordnet sind (Anspruch 1).

Dabei erlaubt die längliche Durchtrittsöffnung, die sich gemäß einem weiteren Ausführungsbeispiel der Erfindung zumindest bereichsweise auf die wenigstens zwei Mittel zum Positionieren des medizinischen Hilfsmittels erstreckt, eine Formgebung des medizinischen Hilfsmittels, d.h. das vorzugsweise schlauchförmige medizinische Hilfsmittel erfährt allein durch die im Trägerelement angeordnete längliche Durchtrittsöffnung eine Krümmung. Dadurch, dass sich die Durchtrittsöffnung zumindest bereichsweise auf die wenigstens zwei Mittel zum Positionieren des medizinischen Hilfsmittels erstreckt, wird verhindert, dass das Trägerelement vom gekrümmten medizinischen Hilfsmittel von der Haut des Patienten weggedrückt und abgelöst wird.

Gemäß einem ersten Ausführungsbeispiel der Erfindung sind dabei die wenigstens zwei Mittel zum Positionieren des medizinischen Hilfsmittels mit dem Trägerelement vorzugsweise einstückig und auf seiner Unterseite nicht klebend ausgebildet, sodass medizinische Hilfsmittel im Gebrauchszustand der Fixiervorrichtung im Wesentlichen einem Abschnitt des Trägerelementes fixiert wird, der sich in unmittelbarer Nähe des Endbereiches der länglichen Durchtrittsöffnung befindet und der auf seiner Unterseite nicht klebend ausgebildet ist.

Um sicherzustellen, dass das medizinische Hilfsmittel auch bei einer Bewegung des Patienten oder bei Zug am medizinischen Hilfsmittel nicht verrutscht, sieht ein weiteres Ausführungsbeispiel der Erfindung vor, dass wenigstens ein Mittel zum Positionieren des medizinischen Hilfsmittels mit dem Trägerelement vorzugsweise einstückig und auf seiner Oberseite zumindest bereichsweise klebend ausgebildet ist.

Weiters hat es sich für einen sicheren Halt des Trägerelementes an der Haut des Patienten sowie zum Definieren der Fixierposition des medizinischen Hilfsmittels als günstig erwiesen, wenn die wenigstens zwei Mittel zum Positionieren des medizinischen Hilfsmittels mit dem Trägerelement vorzugsweise einstückig ausgebildet und vorzugsweise entlang mindestens einer Sollknicklinie klappbar sind, wobei ein bevorzugtes Ausführungsbeispiel der Erfindung vorsieht, dass wenigstens eine Sollknicklinie innerhalb der Fläche des Trägerelementes angeordnet ist und/oder die Durchtrittsöffnung für das medizinische Hilfsmittel quert.

Dadurch wird erreicht, dass die Mittel zum Fixieren des medizinischen Hilfsmittels im Gebrauchszustand der Fixiervorrichtung, d.h. wenn die Mittel zum Fixieren des medizinischen Hilfsmittels bereits in eine zur Ebene des Trägerelementes verschiedene Ebene geklappt bzw. geschwenkt wurden, eine Durchtrittsöffnung für das medizinische Hilfsmittel aufweisen, die eine Verlängerung der am Trägerelement angeordneten Durchtrittsöffnung darstellt. Somit wird erreicht, dass das im Wesentlichen senkrecht aus der Körperoberfläche des Patienten durch das Trägerelement austretenden medizinische Hilfsmittel im Wesentlichen parallel zur Körperoberfläche des Patienten weggeführt werden kann, ohne dass das medizinische Hilfsmittel die Ebene des Trägerelementes noch einmal durchdringen muss.

Gleichzeitig wird durch die sich über das Trägerelement und das aufgeklappte Mittel zum Fixieren des medizinischen Hilfsmittels reichende längliche Durchtrittsöffnung erreicht, dass das medizinische Hilfsmittel in gekrümmtem Zustand in einfacher Weise zweimal durch die eine Durchtrittsöffnung geführt werden kann, wodurch dem medizinischen Hilfsmittel bereits die gewünschte Form gegeben wird. Im Unterschied dazu war es bei den aus dem Stand der Technik bekannten Fixiervorrichtungen notwendig, das medizinische Hilfsmittel durch eine im Trägerelement angeordnete Durchtrittsöffnung durchzuführen und das medizinische Hilfsmittel in der gewünschte Form mittels weiterer Komponenten wie beispielsweise Heftpflaster oder Aufsatzkamponenten mit Führungsrillen festzulegen.

Ein bevorzugtes Ausführungsbeispiel, das eine einfache Handhabung der erfindungsgemäßen Fixiervorrichtung ermöglicht, sieht vor, dass die wenigstens eine Sollknicklinie zwischen den Endpunkten zweier in Bezug auf die Mittellängsachse der Durchtrittsöffnung vorzugsweise symmetrisch und/oder parallel angeordneter, vom Rand in die Fläche des Trägerelementes reichender Schnittlinien verläuft.

Gemäß einem weiteren, Platz sparenden Ausführungsbeispiel der erfindungsgemäßen Fixiervorrichtung ist vorgesehen, dass wenigstens ein Mittel zum Positionieren des medizinischen Hilfsmittels zwei Abschnitte aufweist, von denen ein erster Abschnitt Innerhalb der Fläche des Trägerelementes angeordnet ist, während der zweite Abschnitt gegenüber dem Trägerelement zumindest bereichsweise seitlich vorsteht, wobei es sich als günstig herausgestellt hat, wenn sich die längliche Durchtrittsöffnung zumindest bereichsweise auf den ersten Abschnitt des Mittels zum Positionieren des medizinischen Hilfsmittel erstreckt.

Ist dabei der erste Abschnitt des Mittels zum Positionieren des medizinischen Hilfsmittels vorzugsweise ausschließlich auf seiner Unterseite klebend ausgebildet und/oder der zweite Abschnitt des Mittels zum Positionieren des medizinischen Hilfsmittels vorzugsweise ausschließlich auf seiner Oberseite klebend ausgebildet, wird eine besonders einfache und kostengünstig herzustellende Fixiervorrichtung erreicht.

Die Mittel zum Positionieren des medizinischen Hilfsmittels relativ zum Trägerelement sind in den Endbereichen der länglichen Durchtrittsöffnung des Trägerelementes angeordnet sind, die von der Eintrittsstelle des medizinischen Hilfsmittels in die Körperöffnung entfernt sind, da das medizinische Hilfsmittel am gegenüber liegenden Endbereich der Durchtrittsöffnung nicht zuletzt durch seine Position in der Körperöffnung bis zu einem gewissen Maße fixiert ist.

Um das Verrutschen des medizinischen Hilfsmittels sicher zu verhindern, ist die erfindungsgemäße Fixiervorrichtung durch ein zweites Mittel zum Positionieren des medizinischen Hilfsmittels gekennzeichnet, wobei es sich als zweckmäßig erwiesen hat, wenn die beiden Mittel zum Positionieren des medizinischen Hilfsmittels in Bezug auf die längliche Durchtrittsöffnung gegenüberliegend angeordnet sind und sich die Durchtrittsöffnung zumindest bereichsweise auf das zweite Mittel zum Positionieren des medizinischen Hilfsmittels erstreckt.

Steht das zweite Mittel zum Positionieren des medizinischen Hilfsmittels gemäß einem weiteren Ausführungsbeispiel der Erfindung gegenüber dem Trägerelement zumindest bereichsweise seitlich vor und ist auf seiner Oberseite zumindest bereichsweise klebend ausgebildet, wird erreicht, dass das medizinische Hilfsmittel im Wesentlichen neben dem Trägerelement in die Körperöffnung eintritt, wodurch ein Verkleben der Eintrittsöffnung durch die klebende Unterseite des Trägerelementes sicher vermieden werden kann.

Ein bevorzugtes Ausführungsbeispiel der Erfindung sieht dabei vor, dass ein Mittel zum Positionieren des medizinischen Hilfsmittels zwei Abschnitte aufweist, wobei sich die Durchtrittsöffnung zumindest bereichsweise auf den ersten Abschnitt des Mittels zum Positionieren des medizinischen Hilfsmittels erstreckt und der zweite Abschnitt vorzugsweise ausschließlich auf seiner Oberseite klebend ausgebildet ist, wobei das medizinische Hilfsmittel besonders dann einfach fixiert werden kann, wenn der erste Abschnitt wenigstens auf seiner Oberseite, vorzugsweise beidseitig, nicht klebend ausgebildet ist.

Ein besonders bevorzugtes und einfach herzustellendes Ausführungsbeispiel einer erfindungsgemäßen Fixiervorrichtung ist gekennzeichnet durch ein Trägerelement mit einer länglichen Durchtrittsöffnung und zwei sich in Bezug auf die längliche Durchtrittsöffnung gegenüberliegende, mit dem Trägerelement einstückig ausgebildete Mittel zum positionieren des medizinischen Hilfsmittels, wobei die Mittel zum Positionieren des medizinischen Hilfsmittels jeweils zwei Abschnitte aufweisen, von denen der jeweils in Bezug auf das Trägerelement äußere Abschnitt vorzugsweise ausschließlich auf seiner Oberseite klebend ausgebildet ist und wobei sich die längliche Durchtrittsöffnung zumindest bereideweise auf die jeweils inneren, auf ihrer Oberseite vorzugsweise nicht klebend ausgebildeten Abschnitte der Mittel zum Positionieren des medizinischen Hilfsmittels erstreckt., wobei es sich für eine einfache Handhabung als günstig herausgestellt hat, wenn der innere Abschnitt des zweiten Mittels zum Positionieren des medizinischen Hilfsmittels seitlich über das Trägerelement vorsteht und auf seiner Unterseite nicht klebend ausgebildet ist, während der innere Abschnitt des ersten Mittels zum Positionieren des medizinischen Hilfsmittels innerhalb der Fläche des Trägerelementes angeordnet ist.

Die Dimensionierung der länglichen Durchtrittsöffnung sollte so gewählt sein, dass verschiedenartige medizinische Hilfsmittel mit unterschiedlichen Durchmessern durch sie hindurch geführt werden können. Insbesondere zum Fixieren von schlauchförmigen Hilfsmitteln, die zweimal durch die Durchtrittsöffnung geführt werden, hat es sich als vorteilhaft herausgestellt, wenn das Verhältnis der Länge der Durchtrittsöffnung zur Breite der Durchtrittsöffnung wenigstens 3 : 1, vorzugsweise wenigstens 5 : 1, beträgt, wobei es sich als besonders günstig herausgestellt hat, wenn das Verhältnis in etwa 10 : 1 beträgt.

Es versteht sich von selbst, dass für die Herstellung des Trägerelementes bzw. der Mittel zum Fixieren des medizinischen Hilfsmittels unterschiedlichste Materialien und Kiebstoffe herangezogen werden können. Ein besonders einfacher Herstellungsprozess kann beispielsweise dann erreicht werden, wenn das Trägerelement und/oder die Mittel zum Fixieren des medizinischen Hilfsmittels von einer einseitig klebenden Folie, vorzugsweise einer PET-Selbstklebefolie, gebildet ist/sind.

Ebenso kann die Ausgestaltung der nicht klebenden Unterseite der medizinischen Hilfsmittel auf unterschiedlichste Art und Weise erfolgen, beispielsweise durch Verwendung einer nicht klebenden Folie. Insbesondere bei der Verwendung von einseitig klebenden Folien hat es sich gemäß einem weiteren Ausführungsbeispiel der Erfindung als günstig herausgestellt, wenn das/die Mittel zum Fixieren des medizinischen Hilfsmittels auf seiner/ihrer Unterseite zumindest bereichsweise mit einer nicht klebenden Folie abgedeckt ist/sind und das/die Mittel zum Fixieren des medizinischen Hilfsmittels auf seiner/ihrer Oberseite zumindest bereichsweise mit einer Klebstoffschicht beschichtet sind.

Die vorbeschriebene erfindungsgemäße Fixiervorrichtung eignet sich insbesondere zum Fixieren eines medizinischen Hilfsmittels, vorzugsweise einer Ober den Mundwinkel in die Mundhöhle eingeführten Sauerstoffsonde, wobei das vorzugsweise schlauchförmige Hilfsmittel in den beiden Endbereichen der länglichen Durchtrittsöffnung am Trägerelement anliegt. Dabei wird das medizinischen Hilfsmittel zweimal durch die eine Durchtrittsöffnung geführt, sodass die erfindungsgemäße Fixiervorrichtung sowohl zum Fixieren bzw. Ankleben des medizinischen Hilfsmittels mittels des klebenden Trägerelementes an die Haut eines Patienten als auch zum Formgeben des medizinischen Hilfsmittels mittels der länglichen Durchtrittsöffnung und des wenigstens eine Mittels zum Positionieren des medizinischen Hilfsmittels dient.

Weitere Einzelheiten der Erfindung und der durch sie erzielten Vorteile ergeben sich aus der nachstehenden Erläuterung der in der Zeichnung dargestellten Ausführungsbeispiele einer erfindungsgemäßen Fixiervorrichtung für medizinische Hilfsmittel. Darin zeigt:
- Fig. 1: eine Draufsicht auf ein erstes Ausführungsbeispiel der Erfindung,
- Fig. 2a u. 2b: eine Draufsicht und in einer Seitenansicht schenrtatisch den Aufbau eines weiteren Ausführungsbeispiel der Erfindung und
- Fig. 3a u. 3b: perspektivisch ein Anwendungsbeispiel einer erfindungsgemäßen Fixiervorrichtung.

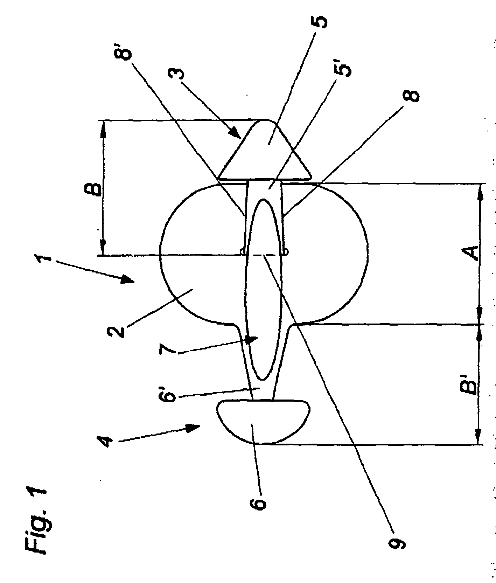

Die in Fig. 1 von oben dargestellte erfindungsgemäße Fixiervorrichtung 1 weist ein Trägerelement 2 sowie zwei mit dem Trägerelement 2 einstückig ausgebildete Mittel 3, 4 zum Fixieren eines medizinischen Hilfsmittels 15 (nicht dargestellt) auf. Das Trägerelement 2 sowie die beiden, jeweils zwei Abschnitte 5, 5', 6, 6' aufweisenden Mittel 3, 4, sind beim gezeigten Ausführungsbeispiel von einer einseitig klebenden PET-Folie gebildet. Die längliche ellipsenförmige Durchtrittsöffnung 7 erstreckt sich vom inneren Abschnitt 6' des zweiten Mittels 4 über das Trägerelement 2 bis auf den inneren Abschnitt 5' des ersten Mittels 3.

Dieser Abschnitt 5' des ersten Mittels 3 ist im wesentlichen zur Gänze innerhalb der Fläche des Trägerelementes 2 angeordnet und wird vom äußeren Abschnitt 5 des Mittels 3 sowie von zwei vom Rand in die Fläche des Trägerelements 2 reichenden Schnitten 8, 8' begrenzt. Die beiden innerhalb des Trägerelementes 2 liegenden Endpunkte der Schnitte 8, 8' bilden gleichzeitig die Endpunkte der Sollknicklinie 9, die im Wesentlichen quer zur Mittellängsachse der Durchtrittsöffnung 7 verläuft und diese schneidet. In ihren Endbereichen sind die Schnitte 8, 8' rund ausgebildet, um ein Einreißen des Trägerelementes 2 bei aufgeklapptem Mittel 3 zu verhindern. Während die Mittel 3, 4 auf der Oberseite ihrer inneren Abschnitte 5', 6' nicht klebend ausgebildet sind, sind die äußeren Abschnitte 5, 6 der Mittel 3, 4 klebend ausgebildet. Zum Fixieren des medizinischen Hilfsmittels werden diese Abschnitte 5, 6 mit ihrer klebenden Fläche um das medizinische Hilfsmittel geschlungen,

Beim gezeigten Ausführungsbeispiel verfügt die erfindungsgemäße Fixiervorrichtung 1 also im Wesentlichen über drei Abschnitte, wobei der mittlere Abschnitt A vom Trägerelement 2 gebildet wird und auf seiner Unterseite klebend ausgebildet ist, während die beiden Abschnitte B, B' von den Mitteln 3, 4 gebildet werden. Dabei überschneiden sich auf Grund der einstückigen Ausbildung der Fixiervorrichtung 1 die Abschnitte A und B.

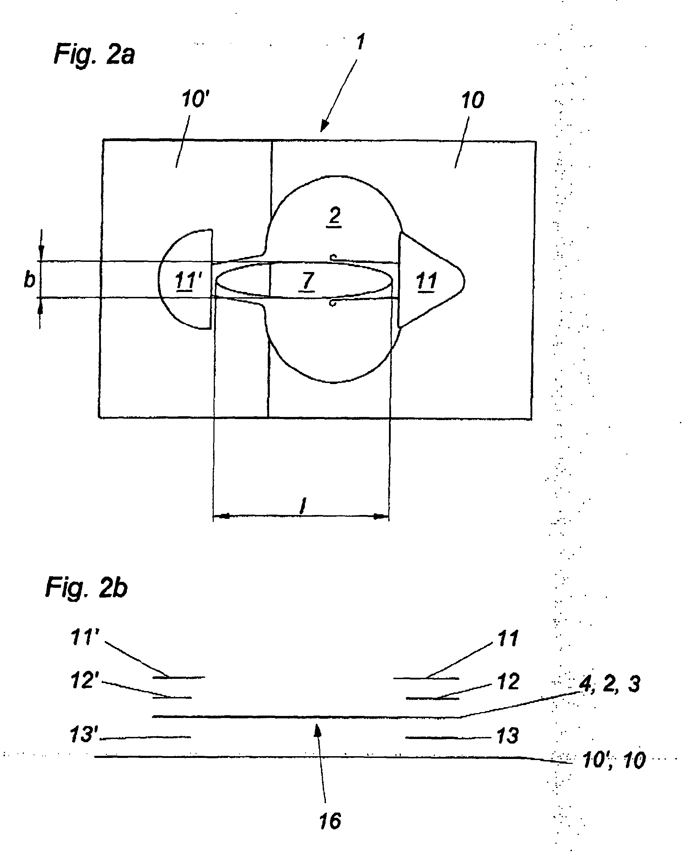

Fig. 2a zeigt im Wesentlichen das Ausführungsbeispiel aus Fig. 1, unterscheldet sich von diesem aber dadurch, dass die klebenden Bereiche auf der Unterseite 16 der Fixiervorrichtung 1 mit einer silikonisierten Abdeckung 10, 10' und die klebenden Bereiche auf der Oberseite ebenfalls mit einer silikonisierten Abdeckung 11, 11' abgedeckt sind. Die zweiteilige Ausführung der unteren Abdeckung 10, 10' soll ein leichtes Entfernen der silikonisierten Abdeckung 10,10' von der Fixiervorrichtung 1 ermöglichen.

Weiters ist aus Fig. 2a ersichtlich, dass die Länge I der Durchtrittsöffnung 7 wesentlich größer ist als die Breite b der Durchtrittsöffnung 7. Erfindungswesentlich ist dabei der Umstand, dass die Durchtrittsöffnung 7 mindestens doppelt so lang wie breit ist, um ein doppeltes Durchführen des medizinischen Hilfsmittels zu ermöglichen.
Fig. 2b zeigt im Prinzip den Aufbau des Ausführungsbeispieles aus Fig. 2a, wobei die Fixiervorrichtung - von unten nach oben betrachtet - eine silikonisierte Abdeckung 10, 10', eine Abdeckschicht 13, 13' zum Deaktivieren der klebenden Unterseite 16 der mit dem Trägerelement 2 einstückig ausgebildeten Mittel 3, 4, eine einseitig klebende PET-Folie 4, 2, 3, eine auf den Mitteln 4, 3 angeordnete Klebstoffschicht 12, 13 und silikonisierte Abdeckungen 11, 11' für die Klebstoffschichten 12, 12' aufweist.

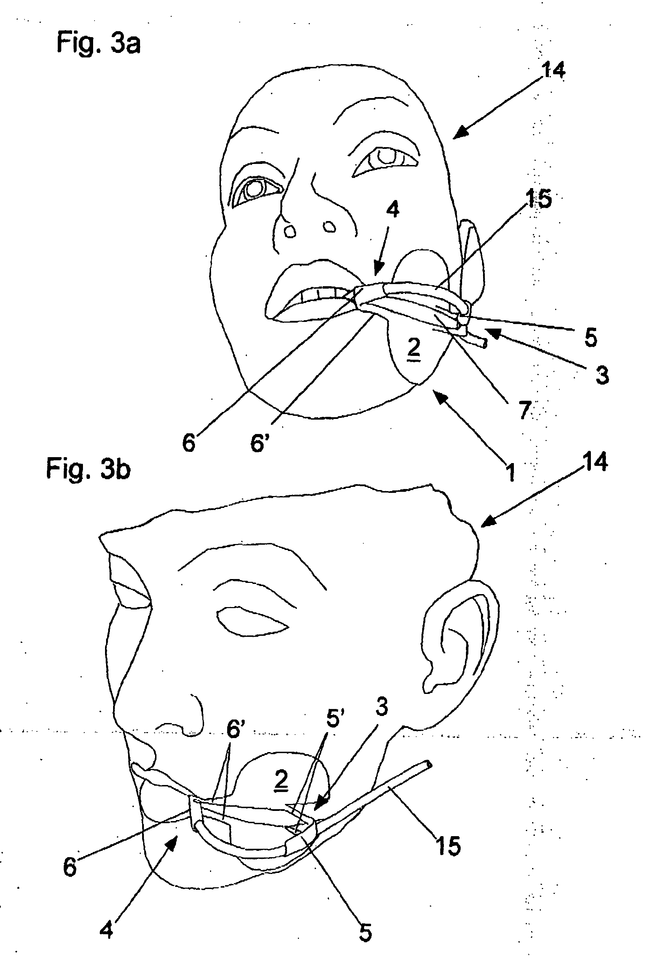

Die Fig. 3a und 3b zeigen perspektivisch eine über den Mundwinkel in die Mundhöhle eines Patienten geführte Sauerstoffsonde, die mittels der Fixiervorrichtung 1 am Kopf 14 eines Patienten fixiert ist. Dabei ist die Fixiervorrichtung 1 mit ihrem Trägerelement 2 an die Wange des Patienten geklebt. Die Sauerstoffsonde ist zweimal durch die Durchtrittsöffnung 7 geführt, wobei die Formgebung des medizinischen Hilfsmittels 15 durch die Mittel 3,4 erfolgt. Die Mittel 3, 4 zum Fixieren des medizinischen Hilfsmittels 15 weisen jeweils zwei Abschnitte 6,6', 5, 5' auf, wobei die jeweils äußeren Abschnitte 6, 5 mit ihren klebenden Seiten um das medizinische Hilfsmittel 15 gewickelt sind und auf diese Weise das medizinische Hilfsmittel 15 fixieren und dessen gekrümmte Form festlegen. Insbesondre aus Fig. 3b ist ersichtlich, dass das medizinische Hilfsmittel im Wesentlichen im rechten Winkel in die Mundhöhle eingeführt ist und auf der gegenüberliegenden Seite der Fixiervorrichtung 1 im Wesentlichen parallel zur Hautoberfläche weggeführt wird. Ermöglicht wird dies dadurch, dass das Mittel 3 aus der Ebene des Trägerelements 2 aufgeklappt ist, wobei sich die Durchtrittsöffnung 7 bis auf den inneren Abschnitt 5' des Mittels 3 erstreckt, wodurch die Fixiervorrichtung 1 nicht von der Spannung des medizinischen Hilfsmittels. 15 von der Wange weggedrückt wird.

Die dargestellten Ausführungsbeispiele von Fixiervorrichtungen von medizinischen Hilfsmittel sowie das beschriebene Verwendungsbeispiel für eine erfindungsgemäße Fixiervorrichtung sind selbstverständlich nicht in einschränkendem Sinne zu verstehen, sondern eben nur einzelne Beispiele von zahlreichen Möglichkeiten deren Erfindungsgedanken von Fixiervorrichtungen für medizinische Hilfsmittel zu verwirklichen.

## Patentansprüche

1. Fixiervorrichtung (1) zum Befestigen eines in eine Körperöffnung einbringbaren vorzugsweise schlauchförmigen medizinischen Hilfsmittels (15) an der Haut eines Subjektes, die ein auf seiner im Gebrauchszustand der Fixiervorrichtung (1) der Haut zugewandten Unterseite (16) zumindest bereichsweise klebend ausgebildetes Trägerelement (2) mit einer länglichen Durchtrittsöffnung (7) aufweist, **dadurch gekennzeichnet dass,** die Fixiervorrichtung (1) wenigstens zwei Mittel (3, 4) zum Positionieren des medizinischen Hilfsmittels (15) relativ zum Trägerelement (2) aufweist, wobei die beiden Mittel (3, 4) zum Positionieren des medizinischen Hilfsmittels (15) in Bezug auf die längliche Durchtrittsöffnung (7) gegenüberliegend in den Endbereichen bezüglich der Längsachse der länglichen Durchtrittsöffnung (7) angeordnet sind.

2. Fixiervorrichtung nach Anspruch 1, **dadurch gekennzeichnet, dass** sich die längliche Durchtrittsöffnung (7) zumindest bereichsweise auf wenigstens ein Mittel (3, 4) zum Positionieren des medizinischen Hilfsmittels (15) erstreckt.

3. Fixiervorrichtung nach Anspruch 1 oder 2, **dadurch gekennzeichnet, dass** wenigstens ein Mittel (3, 4) zum Positionieren des medizinischen Hilfsmittels (15) mit dem Trägerelement (2) vorzugsweise einstückig und auf seiner Unterseite nicht klebend ausgebildet ist.

4. Fixiervorrichtung nach einem der Ansprüche 1 bis 3, **dadurch gekennzeichnet, dass** wenigsten ein Mittel (3, 4) zum Positionieren des medizinischen Hilfsmittels (15) mit dem Trägerelement (2) vorzugsweise einstückig und auf seiner Oberseite zumindest bereichsweise klebend ausgebildet ist.

5. Fixiervorrichtung nach einem der Ansprüche 1 bis 4, **dadurch gekennzeichnet, dass** wenigstens ein Mittel (3, 4) zum Positionieren des medizinischen Hilfsmittels (15) mit dem Trägerelement (2) vorzugsweise einstückig ausgebildet und vorzugsweise entlang mindestens einer Sollknicklinie (9) klappbar ist.

6. Fixiervorrichtung nach Anspruch 5, **dadurch gekennzeichnet, dass** wenigstens eine Sollknicklinie (9) innerhalb der Fläche des Trägerelementes (2) angeordnet ist und/oder die Durchtrittsöffnung (7) für das medizinische Hilfsmittel (15) quert.

7. Fixiervorrichtung nach Anspruch 6, **dadurch gekennzeichnet, dass** die wenigstens eine Sollknicklinie (9) zwischen den Endpunkten zweier in Bezug auf die Mittellängsachse der Durchtrittsöffnung (7) vorzugsweise symmetrisch und/oder parallel angeordneter, vom Rand in die Fläche des Trägerelementes (2) reichender Schnittlinien (8, 8') verläuft.

8. Fixiervorrichtung nach einem der Ansprüche 5 bis 7, **dadurch gekennzeichnet, dass** ein erstes Mittel (3) zum Positionieren des medizinischen Hilfsmittels (15) zwei Abschnitte (5, 5') aufweist, von denen ein erster Abschnitt (5') innerhalb der Fläche des Trägerelementes (2) angeordnet ist, während der zweite Abschnitt (5) gegenüber dem Trägerelement (2) zumindest bereichsweise seitlich vorsteht.

9. Fixiervorrichtung nach Anspruch 8, **dadurch gekennzeichnet, dass** sich die längliche Durchtrittsöffnung (7) zumindest bereichsweise auf den ersten Abschnitt (5') des ersten Mittels (3) zum Positionieren des medizinischen Hilfsmittels (15) erstreckt.

10. Fixiervorrichtung nach Anspruch 8 oder 9, **dadurch gekennzeichnet, dass** der erste Abschnitt (5') des ersten Mittels (3) zum Positionieren des medizinischen Hilfsmittels (15) vorzugsweise ausschließlich auf seiner Unterseite klebend ausgebildet ist und/oder der zweite Abschnitt (5) des ersten Mittels (3) zum Positionieren des medizinischen Hilfsmittels (15) vorzugsweise ausschließlich auf seiner Oberseite klebend ausgebildet ist.

11. Fixiervorrichtung nach einem der Ansprüche 1 bis 10, **dadurch gekennzeichnet, dass** ein zweites Mittel (4) zum Positionieren des medizinischen Hilfsmittels (15) gegenüber dem Trägerelement (2) zumindest teilweise seitlich vorsteht und auf seiner Oberseite zumindest bereichsweise klebend ausgebildet ist.

12. Fixiervorrichtung nach Anspruch 11, **dadurch gekennzeichnet, dass** das zweite Mittel (4) zum Positionieren des medizinischen Hilfsmittels (15) zwei Abschnitte (6, 6') aufweist, wobei sich die Durchtrittsöffnung (7) zumindest bereichsweise auf den ersten Abschnitt (6') des zweiten Mittels (4) zum Positionieren des medizinischen Hilfsmittels (15) erstreckt und der zweite Abschnitt (6) vorzugsweise ausschließlich auf seiner Oberseite klebend ausgebildet ist.

13. Fixiervorrichtung nach Anspruch 12, **dadurch gekennzeichnet, dass** der erste Abschnitt (6') wenigstens auf seiner Oberseite, vorzugsweise beidseitig, nicht klebend ausgebildet ist.

14. Fixiervorrichtung nach einem der Ansprüche 1 bis 13, **gekennzeichnet durch** ein Trägerelement (2) mit einer länglichen Durchtrittsöffnung (7) und zwei sich in Bezug auf die längliche Durchtrittsöffnung (7) gegenüberliegende, mit dem Trägerelement (2) einstückig ausgebildete Mittel (3, 4) zum Positionieren des medizinischen Hilfsmittels (15), wobei die Mittel (3, 4) zum Positionieren des medizinischen Hilfsmittels (15) jeweils zwei Abschnitte (5, 5'; 6, 6') aufweisen, von denen der jeweils in Bezug auf das Trägerelement (2) äußere Abschnitt (5, 6) vorzugsweise ausschließlich auf seiner Oberseite klebend ausgebildet ist und wobei sich die längliche Durchtrittsöffnung (7) zumindest bereichsweise auf die jeweils inneren, auf ihrer Oberseite vorzugsweise nicht klebend ausgebildeten Abschnitte (5', 6') der Mittel (3, 4) zum Positionieren des medizinischen Hilfsmittels (15) erstreckt.

15. Fixiervorrichtung nach Anspruch 14, **dadurch gekennzeichnet, dass** der innere Abschnitt (6') des zweiten Mittels (4) zum Positionieren des medizinischen Hilfsmittels (15) seitlich über das Trägerelement (2) vorsteht und der innere Abschnitt (5') des ersten Mittels (3) zum Positionieren des medizinischen Hilfsmittels (15) innerhalb der Fläche des Trägerelementes (2) angeordnet ist.

16. Fixiervorrichtung nach Anspruch 15, **dadurch gekennzeichnet, dass** der innere, seitlich über das Trägerelement (2) vorstehende Abschnitt (6') des zweiten Mittels (4) zum Positionieren des medizinischen Hilfsmittels (15) auf seiner Unterseite nicht klebend ausgebildet ist.

17. Fixiervorrichtung nach einem der Ansprüche 1 bis 16, **dadurch gekennzeichnet, dass** das Verhältnis der Länge (1) der Durchtrittsöffnung (7) zur Breite (b) der Durchtrittsöffnung (7) wenigstens 3 : 1, vorzugsweise wenigstens 5 : 1, beträgt.

18. Fixiervorrichtung nach Anspruch 17, **dadurch gekennzeichnet, dass** das Verhältnis (1) der Durchtrittsöffnung (7) zur Breite (b) der Durchtrittsöffnung (7) in etwa 10 : 1 beträgt.

19. Fixiervorrichtung nach Anspruch 1 bis 18, **dadurch gekennzeichnet, dass** das Trägerelement (2) und/oder die Mittel (3, 4) zum Fixieren des medizinischen Hilfsmittels (15) von einer einseitig klebenden Folie, vorzugsweise einer PET-Selbstklebefolie, gebildet ist.

20. Fixiervorrichtung nach einem der Ansprüche 1 bis 20, **dadurch gekennzeichnet, dass** das/die Mittel (3, 4) zum Fixieren des medizinischen Hilfsmittels (15) auf seiner/ihrer Unterseite zumindest bereichsweise mit einer nicht klebenden Folie (13, 13') abgedeckt ist/sind.

21. Fixiervorrichtung Anspruch 19 oder 20, **dadurch gekennzeichnet, dass** das/die Mittel (3, 4) zum Fixieren des medizinischen Hilfsmittels (15) auf seiner/ihrer Oberseite zumindest bereichsweise mit einer Klebstoffschicht (12, 12') beschichtet sind.

22. Verwendung einer Fixiervorrichtung nach einem der Ansprüche 1 bis 21 zum Fixieren eines medizinischen Hilfsmittels (15), vorzugsweise einer über den Mundwinkel in die Mundhöhle eingeführten Sauerstoffsonde, wobei das vorzugsweise schlauchförmige Hilfsmittel (15) zweimal durch die eine Durchtrittsöffnung (7) geführt ist und in den beiden Endbereichen der länglichen Durchtrittsöffnung (7) am Trägerelement (2) anliegt.

## Claims

1. A fixing device (1) for fixing a preferably tubular medical accessory (15) which can be introduced into an opening in a body, to the skin of a subject, comprising a carrier element (2) being adhesive at least in a region-wise manner on its underside (16) which in the condition of use of the fixing device (1) is towards the skin, and having an oblongness through opening (7), **characterised in that** the fixing device (1) has at least two means (3, 4) for positioning the medical accessory (15) relative to the carrier element (2), wherein the two means (3, 4) for positioning the medical accessory (15) are arranged in opposite relationship in relation to the oblongness through opening (7) in the end regions with respect to the longitudinal axis of the oblongness through opening (7).

2. A fixing device according to claim 1 **characterised in that** the oblongness through opening (7) extends at least region-wise on to at least one means (3, 4) for positioning the medical accessory (15).

3. A fixing device according to claim 1 or claim 2 **characterised in that** at least one means (3, 4) for positioning the medical accessory (15) is preferably integral with the carrier element (2) and is non-adhesive on its underside.

4. A fixing device according to one of claims 1 to 3 **characterised in that** at least one means (3, 4) for positioning the medical accessory (15) is preferably integral with the carrier element (2) and is at least region-wise adhesive on its top side.

5. A fixing device according to one of claims 1 to 4 **characterised in that** at least one means (3, 4) for positioning the medical accessory (15) is preferably integral with the carrier element (2) and can be folded preferably along at least one predetermined bend line (9).

6. A fixing device according to claim 5 **characterised in that** at least one predetermined bend line (9) is arranged within the surface of the carrier element (2) and/or crosses the through opening (7) for the medical accessory (15).

7. A fixing device according to claim 6 **characterised in that** the at least one predetermined bend line (9) extends between the end points of two section lines (8, 8') which are preferably arranged symmetrically and/or parallel in relation to the longitudinal centre line of the through opening (7) and which extend from the edge into the surface of the carrier element (2).

8. A fixing device according to one of claims 5 to 7 **characterised in that** a first means (3) for positioning the medical accessory (15) has two portions (5, 5'), of which a first portion (5') is arranged within the surface of the carrier element (2) while the second portion (5) at least region-wise laterally projects with respect to the carrier element (2).

9. A fixing device according to claim 8 **characterised in that** the oblongness through opening (7) extends at least region-wise on to the first portion (5') of the first means (3) for positioning the medical accessory (15).

10. A fixing device according to claim 8 or claim 9 **characterised in that** the first portion (5') of the first means (3) for positioning the medical accessory (15) is preferably adhesive exclusively on its underside and/or the second portion (5) of the first means (3) for positioning the medical accessory (15) is preferably adhesive exclusively on its top side.

11. A fixing device according to one of claims 1 to 10 **characterised in that** a second means (4) for positioning the medical accessory (15) at least partially laterally projects with respect to the carrier element (2) and is adhesive at least region-wise on its top side.

12. A fixing device according to claim 11 **characterised in that** the second means (4) for positioning the medical accessory (15) has two portions (6, 6'), wherein the through opening (7) extends at least region-wise on to the first portion (6') of the second means (4) for positioning the medical accessory (15) and the second portion (6) is adhesive preferably exclusively on its top side.

13. A fixing device according to claim 12 **characterised in that** the first portion (13') is non-adhesive at least on its top side, preferably on both sides.

14. A fixing device according to one of claims 1 to 13 **characterised by** a carrier element (2) having an oblongness through opening (7) and two means (3, 4) for positioning the medical accessory (15), which are disposed in opposite relationship with respect to the oblongness through opening (7) and which are formed integrally with the carrier element (2), wherein the means (3, 4) for positioning the medical accessory (15) respectively have two portions (5, 5'; 6, 6') of which the portion (5, 6) which is the respective outer portion with respect to the carrier clement (2) is preferably adhesive exclusively on its top side and wherein the oblongness through opening (7) extends at least region-wise on to the respective inner portions (5', 6'), which are preferably non-adhesive on the top side thereof, of the means (3, 4) for positioning the medical accessory (15).

15. A fixing device according to claim 14 **characterised in that** the inner portion (6') of the second means (4) for positioning the medical accessory (15) projects laterally beyond the carrier element (2) and the inner portion (5') of the first means (3) for positioning the medical accessory (15) is arranged within the surface of the carrier element (2).

16. A, fixing device according to claim 15 **characterised in that** the inner portion (6'), projecting laterally beyond the carrier element (2), of the second means (4) for positioning the medical accessory (15) is non-adhesive on its underside

17. A fixing device according to one of claims 1 to 16 **characterised in that** the ratio of the length (1) of the through opening (7) to the width (b) of the through opening (7) is at least 3:1, preferably at least 5:1.

18. A fixing device according to claim 17 **characterised in that** the ratio (1) of the through opening (7) to the width (b) of the through opening (7) is approximately 10:1.

19. A fixing device according to claims 1 to 18 **characterised in that** the carrier element (2) and/or the means (3, 4) for fixing the medical accessory (15) is formed by a film which is adhesive on one side, preferably a PET self-adhesive film.

20. A fixing device according to one of claims 1 to 20 **characterised in that** the one or more means (3, 4) for fixing the medical accessory (15) is/are covered at least region-wise on its/their underside with a non-adhesive film (13, 13').

21. A fixing device according to claim 19 or claim 20 **characterised in that** the one or more means (3, 4) for fixing the medical accessory (15) is coated on its/their underside at least region-wise with an adhesive layer (12, 12').

22. Use of a fixing device according to one of claims 1 to 21 for fixing a medical accessory (15), preferably an oxygen tube which is introduced into the oral cavity by way of the corner of the mouth, wherein the preferably tubular accessory (15) is guided twice through the one through opening (7) and bears against the carrier element (2) in the two end regions of the oblongness through opening (7).

## Revendications

1. Dispositif de fixation (1) pour la fixation d'un moyen auxiliaire (15) médical, de préférence en forme de flexible, pouvant être introduit dans une ouverture du corps, sur la peau d'un sujet, qui présente un élément support (2) conçu adhésif au moins par endroits sur son côté inférieur (16) tourné vers la peau lorsque le dispositif de fixation (1) est dans l'état d'utilisation, avec une ouverture de passage (7) allongée, **caractérisé en ce que** le dispositif de fixation (1) présente au moins deux moyens (3, 4) pour le positionnement du moyen auxiliaire (15) médical par rapport à l'élément support (2), les deux moyens auxiliaires (3, 4) étant disposés pour le positionnement du moyen auxiliaire (15) médical par rapport à l'ouverture de passage (7) allongée de façon opposée dans les zones d'extrémité par rapport à l'axe longitudinal de l'ouverture de passage (7) allongée.

2. Dispositif de fixation selon la revendication 1, **caractérisé en ce que** l'ouverture de passage (7) allongée s'étend au moins par endroits sur au moins un moyen (3, 4) pour le positionnement du moyen auxiliaire (15) médical.

3. Dispositif de fixation selon la revendication 1 ou 2, **caractérisé en ce qu'**au moins un moyen (3, 4) pour le positionnement du moyen auxiliaire (15) médical est conçu de préférence d'une seule pièce avec l'élément support (2) et n'est pas conçu adhésif sur son côté inférieur.

4. Dispositif de fixation selon l'une quelconque des revendications 1 à 3, **caractérisé en ce qu'**au moins un moyen (3, 4) pour le positionnement du moyen auxiliaire (15) médical est conçu de préférence d'une seule pièce avec l'élément support (2) et est adhésif au moins par endroits sur un côté supérieur.

5. Dispositif de fixation selon l'une quelconque des revendications 1 à 4, **caractérisé en ce qu'**au moins un moyen (3, 4) pour le positionnement du moyen auxiliaire (15) médical est conçu de préférence d'une seule pièce avec l'élément support (2) et peut être plié de préférence le long d'au moins une ligne de pliure théorique (9).

6. Dispositif de fixation selon la revendication 5, **caractérisé en ce qu'**au moins une ligne de pliure théorique (9) est disposée à l'intérieur de la surface de l'élément support (2) et/ou traverse l'ouverture de passage (7) pour le moyen auxiliaire (15) médical.

7. Dispositif de fixation selon la revendication 6, **caractérisé en ce que** la au moins une ligne de pliure théorique (9) passe entre les points d'extrémité de deux lignes de coupe (8, 8') disposée de préférence de façon symétrique et/ou parallèle par rapport à l'axe médian longitudinal de l'ouverture de passage (7) et allant du bord dans la surface de l'élément support (2).

8. Dispositif de fixation selon l'une quelconque des revendications 5 à 7, **caractérisé en ce qu'**un premier moyen (3) pour le positionnement du moyen auxiliaire (15) médical présente deux parties (5, 5'), dont une première partie (5') est disposée à l'intérieur de la surface de l'élément support (2), alors que la seconde partie (5) dépasse latéralement au moins par endroits par rapport à l'élément support (2).

9. Dispositif de fixation selon la revendication 8, **caractérisé en ce que** l'ouverture de passage (7) allongée s'étend au moins par endroits sur la première partie (5') du premier moyen (3) pour le positionnement du moyen auxiliaire (15) médical.

10. Dispositif de fixation selon la revendication 8 ou 9, **caractérisé en ce que** la première partie (5') du premier moyen (3) pour le positionnement du moyen auxiliaire (15) médical est conçue adhésive de préférence uniquement sur son côté inférieur et/ou la seconde partie (5) du premier moyen (3) pour le positionnement du moyen auxiliaire (15) médical est conçue adhésive de préférence seulement sur son côté supérieur.

11. Dispositif de fixation selon l'une quelconque des revendications 1 à 10, **caractérisé en ce qu'**un second moyen (4) pour le positionnement du moyen auxiliaire (15) médical dépasse latéralement au moins partiellement par rapport à l'élément support (2) et est conçu adhésif au moins par endroits sur son côté supérieur.

12. Dispositif de fixation selon la revendication 11, **caractérisé en ce que** le second moyen (4) pour le positionnement du moyen auxiliaire (15) médical présente deux parties (6, 6'), l'ouverture de passage (7) s'étendant au moins par endroits sur la première partie (6') du second moyen (4) pour le positionnement du moyen auxiliaire (15) médical et la seconde partie (6) étant conçue adhésive de préférence uniquement sur son côté supérieur.

13. Dispositif de fixation selon la revendication 12, **caractérisé en ce que** la première partie (6') n'est pas conçue adhésive au moins sur son côté supérieur, de préférence sur les deux côtés.

14. Dispositif de fixation selon l'une quelconque des revendications 1 à 13, **caractérisé par** un élément support (2) avec une ouverture de passage (7) allongée et deux moyens (3, 4) conçus d'une seule pièce avec l'élément support (2) et se faisant face par rapport à l'ouverture de passage (7) allongée, pour le positionnement du moyen auxiliaire (15) médical, les moyens (3, 4) pour le positionnement du moyen auxiliaire (15) médical présentant chacun deux parties (5, 5' ; 6, 6'), dont la partie (5, 6) extérieure par rapport à l'élément support (2) est conçue adhésive de préférence uniquement sur son côté supérieur et l'ouverture de passage (7) allongée s'étendant au moins par endroits sur les parties (5', 6'), respectivement intérieures, de préférence conçues non adhésives sur leur face supérieure, des moyens (3, 4) pour le positionnement du moyen auxiliaire (15) médical.

15. Dispositif de fixation selon la revendication 14, **caractérisé en ce que** la partie (6') intérieure du second moyen (4) pour le positionnement du moyen auxiliaire (15) médical dépasse latéralement de l'élément support (2) et la partie (5') intérieure du premier moyen (3) pour le positionnement du moyen auxiliaire (15) médical est disposée à l'intérieur de la surface de l'élément support (2).

16. Dispositif de fixation selon la revendication 15, **caractérisé en ce que** la partie (6') intérieure, dépassant latéralement de l'élément support (2), du second moyen (4) pour le positionnement du moyen auxiliaire (15) médical est conçue non adhésive sur son côté inférieur.

17. Dispositif de fixation selon l'une quelconque des revendications 1 à 16, **caractérisé en ce que** le rapport de la longueur (1) de l'ouverture de passage (7) à la largeur (b) de l'ouverture de passage (7) est d'au moins 311, de préférence au moins 5/1.

18. Dispositif de fixation selon la revendication 17, **caractérisé en ce que** le rapport (1) de l'ouverture de passage (7) à la largeur (b) de l'ouverture de passage (7) est d'environ 1011.

19. Dispositif de fixation selon les revendications 1 à 18, **caractérisé en ce que** l'élément support (2) et/ou les moyens (3, 4) pour la fixation du moyen auxiliaire (15) médical est/sont formé(s) par un film collant d'un côté, de préférence un film autocollant en polyétylène.

20. Dispositif de fixation selon l'une quelconque des revendications 1 à 20, **caractérisé en ce que** le/les moyen(s) (3, 4) pour la fixation du moyen auxiliaire (15) médical est/sont recouvert(s) sur son/leur côté inférieur au moins par endroits avec un film (13, 13') non adhésif.

21. Dispositif de fixation selon la revendication 19 ou 20, **caractérisé en ce que** le/les moyen(s) (3, 4) pour la fixation du moyen auxiliaire (15) médical est/sont revêtu(s) sur son/leur côté supérieur au moins par endroits d'une couche de colle (12, 12').

22. Utilisation d'un dispositif de fixation selon l'une quelconque des revendications 1 à 21 pour la fixation d'un moyen auxiliaire (15) médical, de préférence d'une sonde d'oxygène introduite par la commissure des lèvres dans la cavité buccale, le moyen auxiliaire (15) de préférence en forme de flexible étant guidé deux fois à travers l'une des ouvertures de passage (7) et s'appuyant dans les deux zones d'extrémité de l'ouverture de passage (7) allongée sur l'élément support (2).
